Europäisches Patentamt

European Patent Office

Office européen des brevets

(19)

(11) Numéro de publication : **0 494 834 A1**

(12) **DEMANDE DE BREVET EUROPEEN**

(21) Numéro de dépôt : **92420005.8**

(22) Date de dépôt : **06.01.92**

(51) Int. Cl.⁵ : **A61B 5/05, A61B 17/36**

(30) Priorité : **11.01.91 FR 9100464**

(43) Date de publication de la demande :
**15.07.92 Bulletin 92/29**

(84) Etats contractants désignés :
**AT BE CH DE DK ES GB GR IT LI LU NL SE**

(71) Demandeur : **DIXWELL**
**Z.I. du Pontet**
**F-69360 St. Symphorien d'Ozon (FR)**

(72) Inventeur : **Aguettant, Jean**
**12 rue Nicolas de Lange**
**F-69005 Lyon (FR)**
Inventeur : **Petitjean, Philippe**
**4 rue des Blés d'Or**
**F-69124 Colombier Saugnieu (FR)**

(74) Mandataire : **Bratel, Gérard et al**
**Cabinet GERMAIN & MAUREAU B.P. 3011**
**F-69392 Lyon Cédex 03 (FR)**

(54) **Procédé et dispositif de mesure de l'impédance de corps biologiques.**

(57) Le procédé et le dispositif s'appliquent plus particulièrement au refroidissement jusqu'à congélation d'un corps biologique (1), avec mesure d'impédance électrique par une pluralité de paires d'électrodes (2) implantables sur le corps (1), fournissant une indication de l'état de congélation de ce corps. Les mesures d'impédance sont réalisées simultanément sur toutes les paires d'électrodes (2) utilisées, grâce à un ensemble de modules de mesure (4) dont chacun correspond à une paire d'électrodes. Les résultats des mesures réalisées sur les différentes paires d'électrodes (2) sont visualisés de façon simultanée et instantanée, par exemple au moyen de rampes de diodes électroluminescentes (6) prévues sur chaque module (4), ce qui donne à tout moment une "vue d'ensemble" de l'état de congélation.
Application au domaine de la cryochirurgie.

FIG.1

EP 0 494 834 A1

La présente invention concerne un procédé perfectionné de mesure de l'impédance électrique de corps biologiques, ainsi qu'un dispositif électronique pour la mise en oeuvre de ce procédé. Le procédé et le dispositif, objets de cette invention, s'appliquent plus particulièrement au refroidissement jusqu'à congélation d'un corps biologique, notamment dans le domaine de la cryochirurgie, avec mesure d'impédance par une pluralité de paires d'électrodes implantables sur le corps considéré, et visualisation des résultats des mesures donnant une indication de l'état de congélation de ce corps.

La cryochirurgie est un procédé de destruction tissulaire "in situ", par congélation localisée. Cette congélation peut être réalisée par divers procédés, qui se différencient notamment par la source frigorigène utilisée : azote liquide, protoxyde d'azote, anhydride carbonique,...

Il est essentiel pour le clinicien de contrôler l'étendue de la cryolésion induite et de délimiter au mieux le volume "cible", afin de réduire à une marge acceptable l'extension de la lésion aux tissus voisins constitués de cellules saines.

Les procédés de contrôle utilisés dans la pratique cryochirurgicale font souvent appel à un principe thermique, avec mesure de température, par implantation d'un ou plusieurs thermocouples ; il s'agit d'un principe simple, mais celui-ci s'avère insuffisamment fiable.

L'utilisation d'une méthode électrique permet d'apprécier, avec un degré de fiabilité élevé, l'état de congélation d'un corps biologique. Une telle méthode repose, de façon générale, sur la mesure de la résistance ou de l'impédance électrique du corps considéré.

En effet, dans un milieu biologique, la résistance électrique du milieu augmente avec le niveau de congélation. Cette résistance reste mesurable aussi longtemps que le milieu considéré présente des veines liquidiennes (l'abaissement de température nécessaire à la disparition totale des veines liquidiennes dépendant de l'état salin du milieu).

La mesure de la résistance électrique d'un tissu biologique peut se faire soit par le passage d'un courant continu, soit par le passage d'un courant alternatif. La mesure par le courant électrique continu impose l'utilisation d'un courant important, d'où un temps de mesure souvent trop court, imposé afin d'éviter la polarisation des électrodes. L'utilisation du courant alternatif implique une mesure non pas de la résistance pure, mais de l'impédance d'un tissu, combinant la résistance et la réactance du tissu, avec pour avantage d'éviter toute interférence liée à la polarisation des électrodes. D'une façon générale, la mesure de l'impédance des tissus biologiques vivants, périphériques ou profonds, par application de la loi d'Ohm, est connue depuis longtemps en médecine.

Cette mesure d'impédance constitue maintenant une méthode reconnue dans le domaine médical, appliquée à des fins de diagnostic et/ou de contrôle. A titre d'exemples particuliers d'applications, on peut citer le phéthysmographe à impédance et la mesure des impédances cutanées pour l'évaluation des cryolésions.

Les dispositifs impédancemétriques actuels, notamment utilisés en dermatologie, permettent d'effectuer des mesures d'impédance par exemple sur le pourtour d'une tumeur que l'on veut détruire par cryochirurgie. La tumeur est entourée par un nombre "n" de paires d'électrodes, ce nombre "n" étant déterminé par la taille de la tumeur et pouvant être compris entre un minimum de une paire et un maximum égal à huit paires d'électrodes ; les électrodes sont implantées dans le corps à une profondeur préalablement déterminée.

La mesure d'impédance est visualisée sur un afficheur mais dans les réalisations actuellement connues, l'information ainsi transmise au clinicien ne concerne à un instant donné qu'une seule paire d'électrodes. Il est nécessaire d'agir manuellement sur un dispositif de commutation, tel que rotacteur ou autre, pour mettre successivement en connexion électrique chaque paire d'électrodes avec un générateur de courant alternatif et avec une tête de mesure de l'impédance.

Les procédés et dispositifs actuels, évoqués ci-dessus, sont décrits dans le brevet français N° 2 289 157 et, encore plus particulièrement, dans le certificat d'addition N° 2 333 483 rattaché à ce brevet. On peut aussi se référer à leurs équivalents étrangers, tels que DE-A-2548262.

Si le choix de la mesure de l'impédance par une ou plusieurs paires d'électrodes, telle qu'enseignée par ces documents, constitue le principe de mesure le plus intéressant, il convient de noter que les dispositifs décrits dans ces documents ne satisfont pas aux besoins actuels. En particulier, en raison du système retenu de commutation avec mesures successives, le clinicien ne peut réaliser une mesure simultanée pour toutes les paires d'électrodes, qui fournirait par l'intermédiaire d'un affichage approprié et à tout instant une "vue d'ensemble" fidèle du degré et de l'étendue de la congélation. Bien au contraire, la mesure réalisée avec l'appareil reste forcément partielle. De plus, l'utilisation de l'appareil implique une action manuelle du praticien ou d'un assistant sur les moyens de commutation, ce qui est contraignant. De plus, une éventuelle mise en mémoire des mesures obtenues avec un processus de commutation manuelle, en vue d'un affichage simultané ultérieur, ne saurait répondre au problème posé car le résultat ne serait pas donné de façon instantanée, en "temps réel".

La présente invention vise à éliminer ces inconvénients, en fournissant un procédé et un dispositif de mesure de l'impédance de corps biologiques qui, quel que soit le nombre de paires d'électrodes, permet de visualiser en permanence et instantanément l'état de congélation dans son ensemble, tout en supprimant les moyens de commutation et les contraintes d'utilisation liées à la commutation.

A cet effet, l'invention a essentiellement pour objet un procédé de mesure de l'impédance de corps biologiques, applicable plus particulièrement au refroidissement jusqu'à congélation d'un corps biologique dans le domaine de la cryochirurgie, avec mesure d'impédance par une pluralité de paires d'électrodes implantables sur le corps considéré, et visualisation des résultats des mesures, le procédé étant caractérisé en ce que les mesures d'impédance sont réalisées simultanément sur toutes les paires d'électrodes utilisées, et en ce que les résultats des mesures ainsi réalisées sur les différentes paires d'électrodes sont visualisés de façon simultanée et instantanée.

De préférence, les mesures d'impédance sont réalisées de manière permanente sur les différentes paires d'électrodes, et la visualisation des résultats des mesures ainsi réalisées est aussi permanente.

L'invention fournit donc un procédé qui permet la mesure "en continu" dans le temps d'une pluralité d'impédances, et qui autorise une indication permanente de l'ensemble des mesures en cours, donnant à tout moment et sans aucune manipulation une "vue d'ensemble" de l'état de congélation dans une zone à surveiller, ceci en "temps réel".

L'invention a aussi pour objet un dispositif spécialement adapté pour la mise en oeuvre du procédé défini précédemment ; il s'agit donc d'un appareil permettant la mesure de l'impédance simultanément sur "n" paires d'électrodes, implantées suivant une disposition adaptée à la zone à surveiller, et l'affichage simultané des "n" mesures réalisées.

Plus particulièrement, le dispositif de mesure selon l'invention comprend un ensemble de modules de mesure, dont chacun correspond à une paire d'électrodes, les modules étant groupés dans un boîtier de commande commun mais restant électriquement isolés les uns des autres, chaque module de mesure comprenant des moyens de génération d'un courant alternatif apte à être injecté dans la paire d'électrodes correspondante, et des moyens de traitement d'un signal électrique représentatif de l'impédance entre les électrodes de la paire considérée, tandis que le boîtier de commande présente sur sa face avant des moyens de visualisation propres respectivement à chaque module de mesure et aptes à fournir une indication de l'impédance mesurée par ce module pour la paire d'électrodes correspondante. Ces moyens de visualisation comprennent par exemple une rampe de diodes électroluminescentes pour chaque module, donc pour chaque paire d'électrodes. On obtient ainsi la simultanéité de mesure et d'affichage pour toutes les paires d'électrodes, avec une bonne "vue d'ensemble" puisque tous les résultats de mesure s'affichent sur la face avant du boîtier, les uns à côté des autres, les "n" modules étant juxtaposés dans ce boîtier et constituant autant de voies de mesure. Des moyens indicateurs complémentaires, notamment de type sonore, peuvent s'ajouter aux moyens de visualisation, pour indiquer qu'une valeur critique est atteinte pour une impédance mesurée.

L'isolation électrique entre les modules permet la simultanéité des mesures, en annulant toute incidence d'une voie de mesure sur l'autre. Cette isolation électrique est obtenue avantageusement par couplage magnétique, notamment en utilisant pour l'alimentation des différents modules en courant alternatif un transformateur multiple ou un même transformateur à plusieurs enroulements secondaires. Une variante prévoit d'utiliser une source de courant continu commune telle que batterie et, pour chaque module de mesure, un convertisseur de tension continu/continu isolé par couplage magnétique.

Le caractère modulaire de la construction proposée permet, par ailleurs, toutes combinaisons en fonction du nombre de paires d'électrodes, et une extension éventuelle du dispositif avec augmentation du nombre de voies de mesure, selon les besoins, par adjonction d'un ou plusieurs modules.

Selon un autre aspect de l'invention, chaque paire d'électrodes forme une sonde de mesure dans laquelle les deux électrodes sont solidarisées et maintenues ainsi parallèles, à un écartement constant prédéterminé, la sonde de mesure étant reliée par un fil ou câble et par un connecteur au module de mesure correspondant. Les deux électrodes ont ainsi le même angle d'implantation sur la région du corps concernée. De préférence, le fil ou câble de liaison est blindé, son blindage étant raccordé à un potentiel "basse impédance" semblable au signal de mesure, ce qui permet d'annuler les capacités parasites du câble ainsi que sa susceptibilité à l'environnement.

De toute façon, l'invention sera mieux comprise à l'aide de la description qui suit, en référence au dessin schématique annexé représentant, à titre d'exemple non limitatif, une forme d'exécution de ce dispositif de mesure de l'impédance de corps biologiques :

Figure 1 est une vue d'ensemble d'un dispositif conforme à la présente invention, en cours d'utilisation ;

Figure 2 montre le détail d'une paire d'électrodes et de ses moyens de liaison avec un module de mesure appartenant au dispositif ;

Figure 3 est une schéma de principe d'un module de mesure.

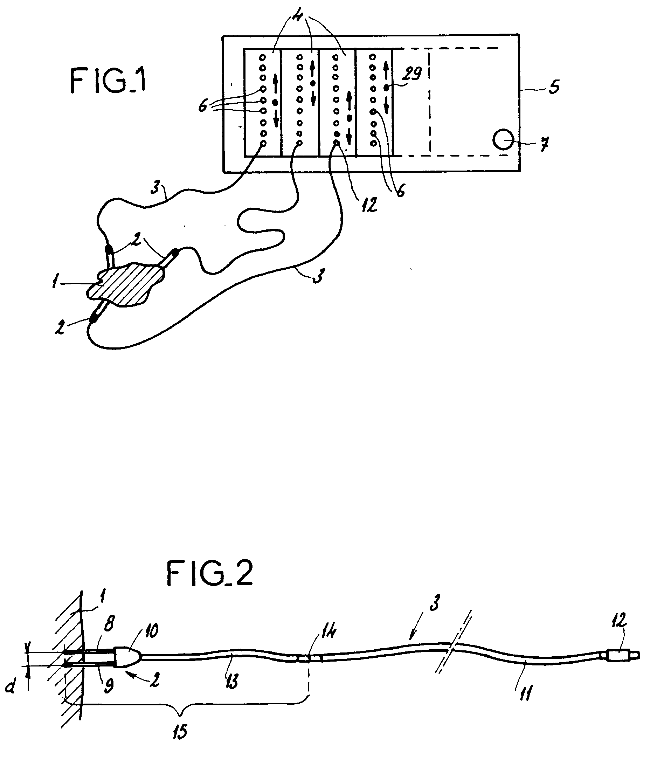

Comme le montre la figure 1, le dispositif objet de l'invention concerne la mesure d'impédance d'un corps biologique, autour d'un volume "cible" 1 tel qu'une tumeur cutanée, soumis à une congélation réalisée elle-même par des moyens connus, non représentés. La mesure d'impédance est multiple et réalisée à l'aide d'une pluralité de paires d'électrodes 2, implantées par exemple en cercle autour du volume 1 à surveiller. Les paires d'électrodes 2 utilisées simultanément dans la même zone sont, par exemple, au nombre de trois.

Chaque paire d'électrodes 2 utilisée est raccordée, par des moyens de liaison indiqués globalement en 3, à un module de mesure 4 associé à cette paire d'électrodes 2. Les modules de mesure 4, correspondant aux différentes paires d'électrodes 2, sont tous similaires et se trouvent groupés dans un boîtier de commande 5 commun, tout en restant isolés les uns des autres d'un point de vue électrique. Chaque module de mesure 4 assure l'injection d'un courant électrique alternatif dans la paire d'électrodes 2 correspondante, et il détermine l'impédance entre les électrodes de la paire 2 considérée.

Le boîtier de commande 5 comporte, dans sa partie arrière, un bloc d'alimentation électrique à voies multiples, pouvant être constitué par un transformateur à enroulements multiples qui assure l'isolation électrique des différents modules de mesure 4 les uns par rapport aux autres. Dans sa partie avant, le boîtier 5 est muni de moyens mécaniques de guidage et de fixation des modules de mesure 4. Ce boîtier 5 reçoit ainsi, par exemple, quatre modules 4 juxtaposés, avec possibilité d'extension à un nombre de modules "n" plus élevé, comme suggéré par le tracé en traits pointillés de la figure 1.

La partie avant du boîtier 5 est au moins partiellement ouverte, pour faire apparaître les faces avant des différents modules de mesure 4. La face avant de chaque module 4 comporte une rampe de diodes électroluminescentes 6, qui représentent des valeurs d'impédance croissantes devant être visualisées. Ainsi, chaque module 4 permet l'affichage de la valeur d'impédance mesurée à l'aide de la paire d'électrodes 2 correspondante.

En cours d'utilisation, un bouton marche-arrêt 7 du boîtier de commande 5 ayant été enclenché, le dispositif peut donc assurer par ses différents modules 4 la mesure et l'affichage simultanés, et permanents, des impédances sur toutes les paires d'électrodes 2 implantées autour du volume 1, ce qui fournit une "vue d'ensemble" de l'état de congélation dudit volume 1.

En se référant à la figure 2, une paire d'électrodes 2 comprend deux électrodes individuelles 8 et 9, sous formes d'aiguilles en acier inoxydable, disposées parallèlement l'une à l'autre à une distance "d" prédéterminée égale à quelques millimètres. L'espacement constant des électrodes 8 et 9 est imposé par un plot 10 surmoulé sur ces électrodes et réalisé en résine synthétique isolante.

Chaque paire d'électrodes 2 forme ainsi une sonde de mesure, reliée à un câble 11 constitué de deux fils blindés (non représentés individuellement) qui aboutit à un connecteur 12 prévu pour le raccordement électrique au module de mesure 4 correspondant, comme le montre aussi la figure 1.

La liaison entre la paire d'électrodes 2 et le câble 11 est réalisée de préférence par l'intermédiaire d'un fil double et souple 13, reliant les deux électrodes 8 et 9 à un connecteur intermédiaire 14. De cette manière, on dispose d'une partie séparable 15, formée par la paire d'électrodes 2 et le fil souple 13, qui peut être stérilisée de façon séparée.

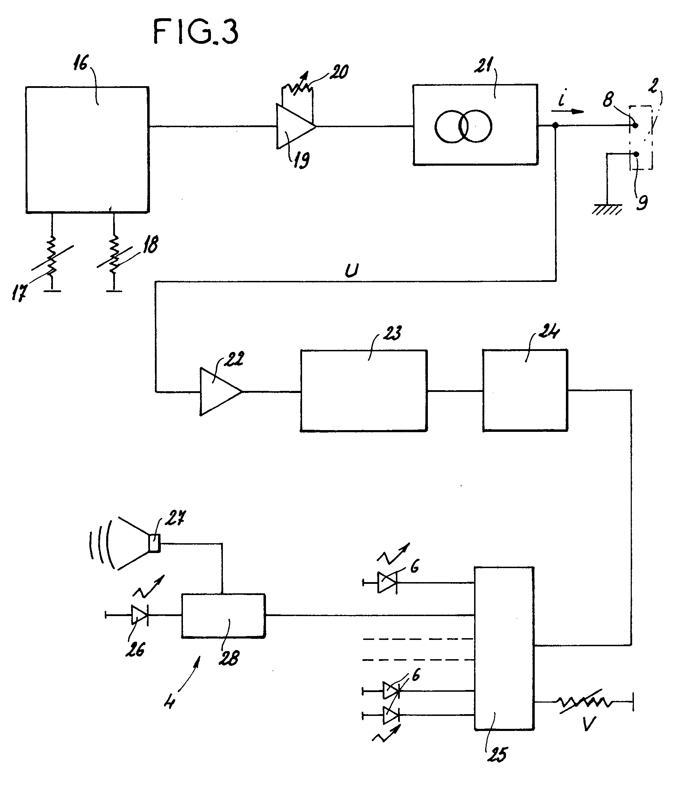

Chaque module de mesure 4 comprend un circuit électronique interne, dont le principe sera maintenant décrit en référence à la figure 3.

Le circuit comprend un générateur d'onde sinusoïdale 16, utilisant un circuit intégré spécifique (par exemple : réf. ICL 8038 de General Electric). Un moyen de réglage de la fréquence 17 et un moyen de réglage de la distorsion 18 sont associés au générateur 16, lequel délivre par exemple un signal possédant une fréquence de l'ordre de 1,5 KHz.

La sortie du générateur d'onde sinusoïdale 16 est reliée à l'entrée d'un amplificateur adaptateur de niveau 19, auquel est associé un organe de réglage de gain 20. Il s'agit d'un amplificateur opérationnel, destiné à imposer l'amplitude de tension du courant sinusoïdal, en fixant par exemple la tension de crête à une valeur de 4 volts.

La sortie de l'amplificateur 19 est reliée à un générateur de courant constant 21, qui permet d'injecter dans la paire d'électrodes 2 de la sonde de mesure associée un courant électrique alternatif maintenu à une intensité "i" connue et maîtrisée, par exemple égale à 7 milliampères, quelle que soit l'impédance à mesurer. De plus, le générateur de courant constant 21 permet de recueillir un signal de mesure "U", qui est la tension aux bornes des électrodes 8 et 9 de la paire d'électrodes 2 considérée, ce signal "U" étant directement proportionnel à l'impédance "Z" à mesurer, à laquelle il est lié par la relation : $U = Z.i$.

Le module de mesure 4 comprend des moyens de traitement du signal de mesure "U", avec un amplificateur adaptateur d'impédance 22 qui recueille ce signal. L'amplificateur adaptateur 22 est caractérisé par une grande impédance d'entrée, prévue pour ne pas perturber la mesure.

La sortie de l'amplificateur adaptateur 22 est reliée à l'entrée d'un redresseur double alternance 23, suivi d'un filtre 24, formant un ensemble de redressement et de filtrage qui transforme le signal de mesure alternatif en un signal analogique continu.

Un convertisseur analogique/numérique 25, recevant une référence de tension "V", transforme enfin sélectivement le signal analogique de mesure en plusieurs états numériques, destinés à exciter successivement les différentes diodes électroluminescentes 6 du module de mesure 4 considéré, formant autant de voyants lumineux fournissant une indication visuelle de la valeur d'impédance mesurée pour la paire d'électrodes 2 considérée.

Le convertisseur analogique/numérique 25 peut comporter une sortie particulière, reliée à des moyens indicateurs complémentaires comprenant une autre diode électroluminescente 26, par exemple clignotante et/ou de couleur particulière, et un ronfleur 27 excité par un oscillateur 28. Un signal lumineux particulier, auquel se superpose un signal sonore, avertit ainsi l'opérateur qu'une valeur de seuil prédéterminée, qui est la valeur critique à ne pas dépasser représentative d'un certain état de congélation, est atteinte au niveau de la paire d'électrodes 2 considérée. Sur chaque module de mesure 4 on peut encore prévoir des moyens, tels qu'un curseur 29, servent à fixer préalablement et à modifier éventuellement la valeur critique précitée de l'impédance mesurée. Le curseur 29 peut être déplaçable en regard des diodes électroluminescentes 6 du module 4 concerné, son positionnement en face de l'une des diodes 6 indiquant que la valeur critique sélectionnée correspond à la valeur d'impédance représentée par cette diode.

Il va de soi que l'invention ne se limite pas à la seule forme d'exécution de ce dispositif de mesure de l'impédance de corps biologiques qui a été décrite ci-dessus, à titre d'exemple ; elle en embrasse, au contraire, toutes les variantes entrant dans le cadre des revendications. C'est ainsi, notamment, que l'on ne s'éloignerait pas du cadre de l'invention par des modifications concernant le nombre des paires d'électrodes simultanément utilisées, la disposition de ces électrodes, la constitution des moyens d'affichage ou d'indication sous toute forme des résultats de mesure, ou le détail du circuit électronique des modules de mesure. Par exemple, les rampés de diodes électroluminescentes sont remplaçables par des échelles graphiques continues, ou par un affichage numérique direct des valeurs d'impédances mesurées.

## Revendications

1. Procédé de mesure de l'impédance électrique de corps biologiques, applicable plus particulièrement au refroidissement jusqu'à congélation d'un corps biologique (1) dans le domaine de la cryochirurgie, avec mesure d'impédance par une pluralité de paires d'électrodes (2) implantables sur le corps (1) considéré, et visualisation des résultats des mesures, caractérisé en ce que les mesures d'impédance sont réalisées simultanément sur toutes les paires d'électrodes (2) utilisées, et en ce que les résultats des mesures ainsi réalisées sur les différentes paires d'électrodes (2) sont visualisés de façon simultanée et instantanée.

2. Procédé de mesure de l'impédance de corps biologiques, selon la revendication 1, caractérisé en ce que les mesures d'impédance sont réalisées de manière permanente sur les différentes paires d'électrodes (2), et en ce que la visualisation des résultats des mesures ainsi réalisées est aussi permanente.

3. Dispositif de mesure de l'impédance de corps biologiques, pour la mise en oeuvre du procédé selon la revendication 1 ou 2, caractérisé en ce qu'il comprend un ensemble de modules de mesure (4), dont chacun correspond à une paire d'électrodes (2), les modules (4) étant groupés dans un boîtier de commande commun (5) mais restant électriquement isolés les uns des autres, chaque module de mesure (4) comprenant des moyens de génération (16 à 21) d'un courant alternatif apte à être injecté dans la paire d'électrodes (2) correspondante, et des moyens de traitement (22 à 25) d'un signal électrique (U) représentatif de l'impédance entre les électrodes (8,9) de la paire considérée, tandis que le boîtier de commande (5) présente sur sa face avant des moyens de visualisation (6) propres respectivement à chaque module de mesure (4) et aptes à fournir une indication de l'impédance mesurée par ce module (4) pour la paire d'électrodes (2) correspondante.

4. Dispositif de mesure de l'impédance de corps biologiques, selon la revendication 3, caractérisé en ce que les moyens de visualisation de l'impédance mesurée, comprennent une rampe de diodes électroluminescentes (6) pour chaque module de mesure (4), donc pour chaque paire d'électrodes (2).

5. Dispositif de mesure de l'impédance de corps biologiques, selon la revendication 4, caractérisé en ce que chaque module de mesure (4) comprend des moyens indicateurs complémentaires (26,27, 28), notamment de type sonore, aptes à indiquer qu'une valeur critique prédéterminée est atteinte pour l'impédance mesurée.

6. Dispositif de mesure de l'impédance de corps biologiques selon la revendication 5, caractérisé en ce que sont encore prévus, sur chaque module de mesure (4), des moyens tels qu'un curseur (29) pour fixer et modifier la valeur critique précitée de l'impédance mesurée.

7. Dispositif de mesure de l'impédance de corps biologiques, selon l'une quelconque des revendications 3 à 6, caractérisé en ce que l'isolation électrique entre les modules de mesure (4) est obtenue par couplage magnétique.

8. Dispositif de mesure de l'impédance de corps biologiques, selon la revendication 7, caractérisé en ce que l'isolation est réalisée par l'utilisation, pour l'alimentation des différents modules de mesure (4) en courant alternatif, d'un transformateur multiple ou d'un même transformateur à plusieurs enroulements secondaires.

9. Dispositif de mesure de l'impédance de corps biologiques, selon la revendication 7, caractérisé en ce que l'isolation est réalisée par l'utilisation, pour l'alimentation des différents modules de mesure (4), d'une source de courant continu commune telle que batterie et, pour chaque module de mesure (4), d'un convertisseur de tension continu/continu isolé par couplage magnétique.

10. Dispositif de mesure de l'impédance de corps biologiques, selon l'une quelconque des revendications 3 à 9, caractérisé en ce que chaque paire d'électrodes (2) forme une sonde de mesure dans laquelle les deux électrodes (8,9) sont solidarisées (en 10) et maintenues ainsi parallèles, à un écartement constant prédéterminé (d), la sonde de mesure étant reliée par un fil ou câble (11) et par un connecteur (12) au module de mesure (4) correspondant.

11. Dispositif de mesure de l'impédance de corps biologiques, selon la revendication 10, caractérisé en ce que le fil ou câble de liaison (11) est blindé, son blindage étant raccordé à un potentiel "basse impédance".

12. Dispositif de mesure de l'impédance de corps biologiques, selon la revendication 10 ou 11, caractérisé en ce que la liaison entre la paire d'électrodes (2) et le fil ou câble (11) est réalisée par l'intermédiaire d'un fil souple (13) et d'un connecteur intermédiaire (14), de manière à disposer d'une partie séparable (15) formée par la paire d'électrodes (2) et le fil souple (13).

FIG.1

FIG.2

# FIG.3

# EP 0 494 834 A1

**Office européen des brevets**

## RAPPORT DE RECHERCHE EUROPEENNE

Numero de la demande

EP    92 42 0005

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | DE-A-2 548 262 (ANVAR) | 1,3,5,11 | A61B5/05 |
| D,Y | * page 7, ligne 1 - ligne 10; figures * | 4 | A61B17/36 |
| | * page 8, ligne 13 - page 11, ligne 18 * | | |
| | --- | | |
| Y | EP-A-0 231 379 (H. MOTOYAMA ET AL.) | 4 | |
| A | * page 5, ligne 10 - page 6, ligne 20 * | 1-3 | |
| | * page 10, ligne 26 - page 13, ligne 25 * | | |
| | * page 18, ligne 8 - page 20, ligne 9 * | | |
| | --- | | |
| A | US-A-4 140 109 (M.I. SAVIC ET AL.) | 1,3,5 | |
| | * colonne 2, ligne 67 - colonne 4, ligne 40 * | | |
| | --- | | |
| A | US-A-3 900 020 (C. LOCK) | 1,3,5 | |
| A | * colonne 3, ligne 11 - colonne 4, ligne 14 * | 7-9 | |
| | * colonne 6, ligne 3 - ligne 17; figures * | | |
| | ----- | | |

|  | DOMAINES TECHNIQUES RECHERCHES (Int. Cl.5) |
|---|---|
| | A61B |

**Le présent rapport a été établi pour toutes les revendications**

| Lieu de la recherche | Date d'achèvement de la recherche | Examinateur |
|---|---|---|
| LA HAYE | 27 MARS 1992 | RIEB K.D. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.............................................................................................
& : membre de la même famille, document correspondant

EPO FORM 1503 03.82 (P0402)

9